(19) Europäisches Patentamt
European Patent Office
Office européen des brevets

(11) EP 0 394 797 B2

(12) # NEUE EUROPÄISCHE PATENTSCHRIFT

(45) Veröffentlichungstag und Bekanntmachung des Hinweises auf die Entscheidung über den Einspruch:
**07.01.1998 Patentblatt 1998/02**

(45) Hinweis auf die Patenterteilung:
**02.06.1993 Patentblatt 1993/22**

(21) Anmeldenummer: **90107180.3**

(22) Anmeldetag: **14.04.1990**

(51) Int. Cl.$^6$: **A61K 6/093**, A61K 6/083

(54) **Dentalmaterial (II)**

Dental material (II)

Matériau dentaire (II)

(84) Benannte Vertragsstaaten:
**AT BE CH DE DK ES FR GB IT LI LU NL**

(30) Priorität: **22.04.1989 DE 3913250**

(43) Veröffentlichungstag der Anmeldung:
**31.10.1990 Patentblatt 1990/44**

(73) Patentinhaber: **DEGUSSA AG**
**60311 Frankfurt (DE)**

(72) Erfinder:
- **Panster, Peter, Dr.**
  **D-6458 Rodenbach (DE)**
- **Janda, Ralf, Dr.**
  **D-6380 Bad Homburg v.d.H. (DE)**
- **Kleinschmit, Peter, Dr.**
  **D-6450 Hanau 9 (DE)**

(56) Entgegenhaltungen:
**EP-A- 0 060 911** **EP-A- 0 287 877**
**EP-A- 0 381 961** **DE-A- 3 416 545**
**DE-A- 4 002 726** **GB-A- 2 051 842**
**GB-A- 2 058 088** **US-A- 4 764 797**

EP 0 394 797 B2

**Beschreibung**

Die Erfindung bezieht sich auf ein pastöses, in Gegenwart eines Initiators zu einer auf Hochglanz polierbaren Masse aushärtbares Dentalmaterial aus einem polymerisierbaren organischen Bindemittel und einem feinteiligen Füllstoff.

Das Material enthält als Bindemittel mindestens ein polymerisierbares Acrylat bzw. Methacrylat und einen gegebenenfalls silanisierbaren neuartigen Füllstoff auf Polysiloxan-Basis. Daneben können Initiatoren zur Polymerisationsauslösung, weitere Füllstoffe wie fein gemahlene Gläser, hochdisperse Kieselsäure oder vorgebildete Polymerisate, Pigmente und Stabilisatoren enthalten sein. Auch andere Additive wie Weichmacher oder Schlagzähigkeitsverbesserer kommen in Frage.

Der Begriff umfaßt beispielsweise Füllungsmaterialien, um kariöse Defekte oder andere Zahndefekte Im Mundraum zu versorgen, Kronen- und Brückenmaterialien, Verblendungen, Versiegelungs- und Schutzüberzugsmassen, Kunststoffbefestigungsmaterialien zum Festsetzen von Inlays oder Kronen und Brücken, Stumpfaufbaumaterialien, Prothesenmaterialien sowie Massen zur Herstellung von künstlichen Zähnen.

Übliche Dentalmassen der obengenannten Art enthalten mindestens einen monomeren Ester der Methacrylsäure, meist aber ein Gemisch aus mehreren solcher Ester. Geeignete monofunktionelle Ester der Methacrylsäure sind beispielsweise Methylmethacrylat, Ethylmethacrylat, Isopropylmethacrylat, n-Hexylmethacrylat und 2-Hydroxyethylmethacrylat.

Neuerdings werden häufig auch mehrfunktionelle Ester der Methacrylsäure mit höherem Molekulargewicht eingesetzt, wie Ethylenglykoldimethacrylat, Butandiol-1,4-dimethacrylat, Triethylenglykoldimethacrylat, Dodecandiol-1,12-dimethacrylat, Decandiol-1,10-dimethacrylat, 2,2-Bis[p(γ-methacryloxy-β-hydroxypropoxy)-phenyl]-propan das Diadukt aus Hydroxyethylmethacrylat und Isophorondiisocyanat, Trimethylolpropantrimethacrylat, Pentaerythrittrimethacrylat, Pentaerythrittetramethacrylat und 2,2-Bis-[p(β-hydroxy-et-hoxy)-phenyl]-propandimethacrylat (Bis-GMA).

Die Materialien für die dentale Anwendung können, je nach Anwendungszweck, auf unterschiedliche Weise ausgehärtet werden.

Zahnfüllmaterialien gibt es sowohl als lichthärtende als auch als selbsthärtende (autopolymerisierende) Massen. Die lichthärtenden Massen enthalten Fotoinitiatoren wie Benzoinalkylether, Benzilmonoketale, Acylphosphinoxide oder aliphatische und aromatische 1,2-Diketo-Verbindungen wie beispielsweise Campherchinon sowie Polymerisationsbeschleuniger wie aliphatische oder aromatische tertiäre Amine (z. B. N,N-Dimethyl-p-toluidin Triethanolamin) oder organische Phosphite und erhärten bei Bestrahlung mit UV- oder sichtbarem Licht.

Die selbsthärtenden Materialien bestehen in der Regel aus einer Katalysator- und einer Basispaste, von denen jede den Bestandteil eines Redoxsystems enthält und die beim Vermischen beider Komponenten polymerisieren. Der eine Bestandteil des Redoxsystems ist meistens ein Peroxid, wie beispielsweise Dibenzoylperoxid, der andere meistens ein tertiäres aromatisches Amin, wie beispielsweise N,N'-Dimethyl-p-toluidin.

Andere Dentalmaterialien wie Prothesenkunststoffe oder Kunststoffmassen zur Herstellung künstlicher Zähne können unter Wärmeeinwirkung polymerisiert werden. Als Initiatoren dienen hieb in der Regel Peroxide wie Dibenzoylperoxid, Dilaurylperoxid oder Bis (2,4-dichlor-benoylperoxid).

Alle Dentalmaterialien enthalten weiterhin in der Regel Pigmente, die - in geringer Menge zugesetzt - dazu dienen, die Farbe der Dentalmassen mit den verschiedenen Schattierungen natürlicher Zähne in Übereinstimmung zu bringen. Geeignete Pigmente sind beispielsweise Eisenoxidschwarz, Eisenoxidrot, Eisenoxidgelb, Eisenoxidbraun Cadmiumgelb und -orange, Zinkoxid und Titandioxid.

Dentalmaterialien enthalten ferner meistorganische oder anorganische Füllstoffe. Dies geschieht, um die Volumenschrumpfung der Kunststoffmasse bei der Polymerisation zu vermindern. Reines monomeres Methylmethacrylat schrumpft beispielsweise bei der Polymerisation um ca. 20 Vol.%. Durch Zusatz von etwa 60 Gewichtsteilen festem Methylmethacrylat-Perlpolymerisat kann die Schrumpfung auf ca. 5 - 7 Vol.% reduziert werden (DE-PS 24 03 211).

Andere organische Füllstoffe werden erhalten, indem man ein Polymerisat herstellt, das im wesentlichen aus Estern der Methacrylsäure besteht und unvernetzt oder vernetzt ist. Gegebenenfalls enthält dieses Polymerisat oberflächenbehandelte Füllstoffe. Ist es als Perlpolymerisat hergestellt, kann es dem Dentalmaterial in dieser Form zugesetzt werden; ist es dagegen durch Substanzpolymerisation in kompakter Form hergestellt, so muß es vor der Einbringung in das Dentalmaterial erst zu einem sog. Splitterpolymerisat vermahlen werden.

Häufig verwendete vorgebildete Polymerisate sind neben den bereits erwähnten füllstoffhaltigen Perl- und Splitterpolymerisaten Homopolymerisate des Methacrylsäuremethylesters oder, vorzugsweise unvernetzte, Copolymerisate des Methacrylsäuremethylesters mit einem geringen Anteil an Estern der Methacrylsäure oder Acrylsäure mit 2 bis 12 C-Atomen in der Alkoholkomponente, zweckmäßigerweise in der Form eines Perlpolymerisates. Andere geeignete Polymerisate sind unvernetzte Produkte auf der Basis von Polyurethanen, Polycarbonaten, Polyestern und Polyethern.

Anorganische Füllstoffe sind beispielsweise gemahlene Gläser oder Quarz mit mittleren Teilchengrößen zwischen etwa 1 und 10 µm sowie hochdisperses $SiO_2$ mit mittleren Teilchengrößen zwischen etwa 10 - 400 nm.

Bei den Gläsern handelt es sich vorzugsweise um Aluminiumsilikatgläser, die mit Barium, Strontium oder seltenen

Erden dotiert sein können (DE-PS 24 58 380).

Hinsichtlich des feingemahlenen Quarzes bzw. der feingemahlenen Gläser sowie des hochdispersen $SiO_2$ bleibt anzumerken, daß der anorganische Füllstoff in der Regel vor dem Vermischen mit den Monomeren zwecks besserer Bindung an die organische Matrix silanisiert wird. Hier für werden die anorganischen Füllstoffe mit Silankupplungsmitteln überzogen, die meistens eine polymerisierbare Doppelbindung zur Reaktion mit den monomeren Estern der Methacrylsäure aufweisen.

Geeignete Silankupplungsmittel sind beispielsweise Vinyltrichlorsilan, Tris-(2-methoxyethoxy)-vinylsilan, Tris-(acetoxy)-vinylsilan und 3-Methacryloyloxy-propyltrimethoxysilan.

Auch die eingangs erwähnten, neuerdings verwendeten Monomere mit höherem Molekulargewicht bewirken ebenfalls eine Verringerung der Polymerisationsschrumpfung. Diesen Monomeren werden nun bis zu etwa 85 Gew.% die beschriebenen inerten anorganischen feingemahlenen Gläser oder organischen Füllstoffe oder Gemische aus diesen zugesetzt, wodurch eine weitere Reduzierung der Schrumpfung auf ca. 1 Vol.% erreicht werden kann.

Die anorganischen Füllstoffe bewirken nicht nur eine Verminderung der Polymerisationsschrumpfung, sondern darüber hinaus auch eine erhebliche Verstärkung des organischen Polymergefüges. Diese Verstärkung macht sich in einer Verbesserung der mechanischen Eigenschaften sowie in einer Erhöhung der Abriebsfestigkeit deutlich bemerkbar (R. Janda, Quintessenz 39, 1067, 1243, 1393, (1988)). Gute mechanische Eigenschaften und hohe Abriebsfestigkeit sind wichtige Forderungen, denen eine Dentalmasse, die verlorene Zahnhartsubstanz dauerhaft ersetzen soll, gerecht werden muß.

Neben den verstärkenden Eigenschaften müssen die Füllstoffe aber ebenfalls auch anderen Materialparametern gerecht werden. Ein wesentlicher Parameter in diesem Zusammenhang ist die Polierbarkeit. Hochglanzpolierbarkeit ist für Füllungsmaterialien und Kronen- und Brückenmaterialien aus mindestens zwei Gründen von erheblicher Bedeutung:

- Aus ästhetischen Gründen ist vom Füllungsmaterial eine hochglänzende und völlig homogene Oberfläche zu fordern, damit die Füllung vom umgebenden, absolut glatten, natürlichen Zahnschmelz nicht mehr zu unterscheiden ist. Weiterhin muß diese hochglänzende Füllungsoberfläche ihren Charakter auch langfristig beibehalten.
- Eine hochglatte Füllungsoberfläche ist auch deshalb wichtig, damit Plaque oder verfärbende Medien keine mechanischen Verankerungsstellen vorfinden.

Nun hat es sich aber gezeigt, daß die vorstehend beschriebenen feingemahlenen Quarz- oder Glasfüllstoffe zwar gute verstärkende Eigenschaften besitzen, jedoch hinsichtlich ihrer Polierbarkeit nicht den Anforderungen entsprechen. Daher hat man versucht, diese anorganischen Füllstoffe noch feiner zu mahlen, um homogenere Oberflächen zu erhalten. Den physikalischen Mahlmethoden sind allerdings Grenzen gesetzt, so daß mittlere Korngrößen unter 1 µm nur noch sehr schwer zu erzeugen sind.

Als man hochdisperse Kieselsäure (mittlere Teilchengröße 10 - 400 nm) als Füllstoff in Dentalmassen verwendete (DE-PS 24 03 211), zeigte sich überraschenderweise, daß mit diesen Füllstoffen eine erhebliche Verbesserung der Polierbarkeit erreicht werden konnte. Nachteile der hochdispersen Kieselsäuren bestehen aufgrund ihrer stark verdickenden Wirkung, so daß heute in der Regel Füllgrade über 52 Gew.% nicht erreicht werden können, es sei denn, man begnügt sich mit unzureichenden verarbeitungstechnischen Eigenschaften.

Darüber hinaus zeigen die mit hochdisperser Kieselsäure gefüllten Materialien deutlich geringere Festigkeiten und Härten als die mit Quarz oder feingemahlenen Gläsern gefüllten.

Aufgabe der Erfindung ist, neue, in Gegenwart eines Initiators härtende, d. h. licht-, heiß- oder selbsthärtende bzw. katalytisch härtende Dentalmaterialien aus einem polymerisierbaren organischen Bindemittel und einem feinteiligen Füllstoff bereitzustellen, die einerseits auf Hochglanz polierbar sind und damit den ästhetischen Anforderungen eines Dentalwerkstoffes genügen, andererseits aber bessere physikalische Eigenschaften besitzen als die den gegenwärtigen Stand der Technik repräsentierenden polierbaren Dentalmaterialien.

Die erfindungsgemäßen Füllstoffe müssen, sofern keine Doppelbindungsfunktionalität gegeben ist, natürlich vor ihrer Einbringung in die organische Polymermatrix mit einer geeigneten Organosilanverbindung, vorzugsweise 3-Methacryloyloxypropyltrimethoxy- oder 3-Methacryloyloxypropyltriethoxysilan, umgesetzt werden. Dies schließt jedoch nicht aus, daß auch Doppelbindungs-funktionelle Füllstoffe noch zusätzlich silanisiert werden.

Analoges gilt auch für eine weitere, aus Gründen der besonders leichten Verfügbarkeit der Ausgangsmaterialien vorteilhafte erfindungsgemäße Füllstoffzusammensetzung, die einen Aufbau des Heterosiloxans aus Einheiten der Formel (I), (II) und den speziellen Einheiten der Formel (IV)

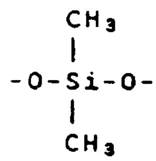

vorsieht, wobei das molare Verhältnis der Summe der Einheiten der Formel (I) und (IV) zu den Einheiten der Formel (II) 1 : 1 bis 100 : 1 beträgt.

Die monomeren Bausteine der erfindungsgemäßen Füllstoffe sind prinzipiell bekannte Verbindungen. Eine Monomerverbindung für eine Einheit der Formel (I) ist z. B.

$$Si(OC_2H_5)_4$$

für eine Einheit der Formel (II) ist z. B.

$$Ti(OC_3H_7)_4$$

für eine Einheit der Formel (III) ist z. B.

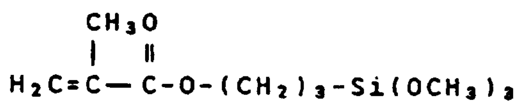

bzw.

$$CH_3\text{-}CH_2\text{-}CH_2\text{-}Si(OCH_3)_3$$

und für eine Einheit der Formel (IV) ist z. B.

$$(CH_3)_2Si(OC_2H_5)_2$$

Die Zusammensetzungen der daraus herstellbaren erfindungsgemäßen Dentalfüllstoffe lassen sich z. B. durch Formeln für eine jeweilige Polymereinheit wie

$$30\ SiO_2 \cdot TiO_2$$

$$40\ SiO_2 \cdot CH_3CH_2CH_2SiO_{3/2} \cdot TiO_2$$

$$20\ SiO_2 \cdot CH_3CH_2CH_2SiO_{3/2} \cdot TiO_2 \cdot (CH_3)_2SiO_{2/2}$$

$$10\ SiO_2 \cdot TiO_2 \cdot (C_2H_5)_2SiO_{2/2}$$

oder

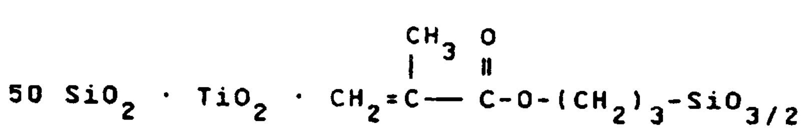

beschreiben.

Im Hinblick auf die physikalischen Eigenschaften besitzen Füllstoffe der erfindungsgemäßen Zusammensetzung dann eine besonders gute Eignung zur Verwendung in den erfindungsgemäßen Dentalmaterialien, wenn sie eine spezifische Oberfläche von ca. 0 bis 200 $m^2/g$, vorzugsweise ca. 0 bis 100 $m^2/g$, und eine Partikelgröße von 0,01 µm bis 100 µm, vorzugsweise 0,1 µm bis 30 µm, aufweisen.

Die in den erfindungsgemäßen Dentalmaterialien enthaltenen Füllstoffe sind nach verschiedenen Methoden erhältlich. Eine davon sieht vor, daß der Füllstoff in Gestalt eines statistischen Polykondensats erhältlich ist, indem man ein Alkoxysilan der allgemeinen Formel

$$Si(OR^3)_4 \qquad (V)$$

wobei $R^3$ für eine lineare oder verzweigte Alkylgruppe mit 1 bis 5 C-Atomen steht, und eine Titanverbindung der allgemeinen Formel

$$Ti(OR^3)_4 \qquad (VI)$$

in der $R^3$ dieselbe Bedeutung wie in Formel (V) hat und gegebenenfalls ein Alkoxysilan derallgemeinen Formel

$$R^1 - Si(OR^3)_3 \qquad (VII)$$

in der $R^1$ für eine lineare oder verzweigte Alkylgruppe mit 1 bis 18 C-Atomen, eine Cycloalkylgruppe mit 5 bis 8 C-Atomen oder eine Arylalkylgruppe oder für eine mit einem Acrylat- oder Methacrylatrest verbundene lineare oder verzweigte Alkylgruppe mit 1 bis 6 C-Atomen oder für einen einfach olefinisch ungesättigten, vorzugsweise endständig ungesättigten linearen oder verzweigten Kohlenwasserstoffrest mit 5 - 8 C-Atomen steht und gegebenenfalls ein Alkoxysilan der allgemeinen Formel

$$R^2_2\, Si(OR^3)_2 \qquad (VIII)$$

in der $R^2$ eine Methyl-, Ethyl- oder Phenylgruppe repräsentiert und $R^3$ in Formel (VII) und (VIII) dieselbe Bedeutung wie in Formel (V) und (VI) hat, in einem weitgehend wassermischbaren, aber die Verbindungen nach Formeln (V) bis (VIII) lösenden Lösungsmittel auflöst und der Lösung unter Rühren eine zumindest für die vollständige Hydrolyse und Kondensation ausreichende Menge Wasser zufügt, dann das Reaktionsgemisch unter Weiterrühren bei einer bestimmten Temperatur im Bereich von Raumtemperatur bis 200° C vorkondensiert, den sich bildenden Feststoff gegebenenfalls nach Zusatz weiteren Lösungsmittels oder Wassers noch 1 Stunde bis zu 6 Stunden bei 60° bis 200° C, bei Normaldruck oder einem Druck rührt, welcher der Summe der Partialdrucke bei der jeweiligen Temperatur entspricht, dann das gebildete Heterosiloxan, gegebenenfalls nach einem Wechsel des Mediums und/oder pH-Wertes noch 4 Stunden bis 5 Tage bei 100° C bis 250° C in flüssiger Phase nachbehandelt, dann nach gängigen Techniken von der flüssigen Phase abtrennt, gegebenenfalls wäscht, bei Raumtemperatur bis 200° C, gegebenenfalls unter Schutzgasatmosphäre oder im Vakuum trocknet, gegebenenfalls 1 bis 100 Stunden bei Temperaturen von 150° C bis 300° C unter Schutzgasatmosphäre oder im Vakuum tempert, gegebenenfalls mahlt und/oder klassifiziert, wobei man den von der flüssigen Phase abgetrennten und gegebenenfalls gewaschenen Heterosiloxan-Füllstoff vor oder nach einer der Stufen Trocknung, Temperung, Mahlung, Klassifizierung in Wasser, einem Wasser/Alkohol-Gemisch oder in reinem Alkohol, in Gegenwart eines sauren oder eines basischen Katalysators, bevorzugt in Gegenwart von Ammo-niak, über einen Zeitraum von 1 Stunde bis zu 5 Tagen bei Temperaturen von 60° C bis 250° C unter einem Druck, welcher der Summe der Partialdrucke bei der jeweiligen Temperatur entspricht, behandelt.

Die vorteilhaften anwendungstechnischen Eigenschaften der neuen Füllstoffe gehen auch auf die saure oder alkalische Temperaturbehandlung vor oder nach Trocknung oder einer der gegebenenfalls noch angewandten Behandlungsstufen zurück, da dadurch vor allem eine Festigung der Polymerstruktur erreicht wird. Ein typischer saurer Katalysator ist z. B. Salzsäure oder Essigsäure, während z. B. Ammoniak oder ein Amin einen typischen Basenkatalysator repräsentieren.

Prinzipiell können als Ausgangsstoffe für das Verfahren anstelle der Alkoxyverbindungen auch die entsprechenden Halogenid- oder Phenoxyverbindungen eingesetztwerden, doch bietet deren Verwendung keine Vorteile, sondern kann z. B. im Fall der Chloride, Schwierigkeiten durch die bei der Hydrolyse freiwerdende HCl verursachen.

Die Hydrolyse der Monomeren muß in einem weitgehend wassermischbaren, aber die Ausgangsstoffe lösenden Lösungsmittel durchgeführt werden. Bevorzugt werden Alkohole verwendet, die zu den Alkoxygruppierungen an den monomeren Ausgangsstoffen korrespondieren. Besonders geeignet sind Methanol, Ethanol, n- und i-Propanol, n- und i-Butanol und n-Pentanol. Auch Gemische solcher Alkohole können als Lösungsmittel bei der Hydrolyse eingesetzt werden. Anstelle von Alkoholen können natürlich auch andere polare Lösungsmittel, die weitgehend wassermischbar sind, verwendet werden, doch erweist sich dies aus verfahrenstechnischen Gründen wegen der mit dem hydrolytisch

abgespaltenen Alkohol zustandekommenden Lösungsmittelgemische als nicht sinnvoll.

Bevorzugt führt man die Hydrolyse mit einem Überschuß an Wasser über die stöchiometrisch erforderliche Menge durch. Die zur Hydrolyse benötigte Menge Wasser hängt von der Hydrolysegeschwindigkeit der jeweils verwendeten Monomeren in der Weise ab, daß mit zunehmender Menge Wasser raschere Hydrolyse erfolgt, allerdings kann eine Obergrenze durch auftretende Entmischung und Ausbildung eines Zweiphasensystems vorgegeben sein. Grundsätzlich ist eine Hydrolyse in homogener Lösung vorzuziehen.

Aufgrund dergenannten Aspekte wird in der Praxis gewichtsmäßig maximal die Menge Wasser verwendet, wie an Silanmonomeren insgesamt eingesetzt wird.

Die Polykondensation kann bei verschiedenen Temperaturen durchgeführt werden. Nachdem die Polykondensation bei höheren Temperaturen am schnellsten verläuft, wird diese bevorzugt bei Rückflußtemperatur oder knapp darunterdurchgeführt. Prinzipiell kann die Hydrolyse und Polykondensation bei noch höherer Temperatur als Rückflußtemperatur, d. h. unter Druck, durchgeführt werden.

Bei der Polykondensation kann das Reaktionsgemisch zu einerfesten Masse erstarren. Aus diesem Grunde ist es angebracht, eine entsprechende Menge Lösungsmittel oder Wasser zur Verdünnung zuzusetzen.

Das Lösungsmittel wird dabei in der Regel dasselbe sein, das schon bei der Hydrolyse der Silane eingesetzt wurde, d. h. bevorzugt wird ein niederer Alkohol mit 1 bis 5 C-Atomen verwendet.

Alternativ zu der Verdünnung mit einem Lösungsmittel kann natürlich auch mit Wasser verdünnt werden. Was im Einzelfall verwendet wird, hängt auch davon ab, welche physikalischen Eigenschaften das herzustellende Copolykondensat haben soll. Auch durch die Dauer und Temperatur der Nachbehandlung in flüssiger Phase oder in trockener Form kann hierauf Einfluß genommen werden. Eine Nachreaktion bei höherer Temperatur führt durchwegs zu einer Verfestigung der Struktur des Polymeren und zu einer Verbesserung der mechanischen Eigenschaften.

Die Abtrennung des gebildeten Feststoffs kann nach gängigen Techniken wie Filtrieren, Dekantieren, Zentrifugieren oder durch Abdestillieren der flüssigen Phase erfolgen. Die Waschung des gebildeten Feststoffs wird bevorzugt mit dem bei der Fällung verwendeten Lösungsmittel oder mit Wasser durchgeführt

Das getrocknete bzw. getemperte Produkt kann in üblichen Vorrichtungen gemahlen und in verschiedene Korngrößenfraktionen klassifiziert werden. Von den Aufarbeitungsmaßnahmen Waschung, Trocknung, Temperung, Mahlung und Klassifizierung kann, je nach Umständen, die eine oder andere entfallen oder in einer anderen Reihenfolge durchgeführt werden.

Eine Klassifizierung kann z. B. auch an flüssigkeitsfeuchtem, gegebenenfalls vorher getrocknetem oder getempertem Produkt durchgeführt werden.

Die Dauer der Hydrolyse hängt von der Hydrolyseeignung der Ausgangsstoffe und von der Temperatur ab. Die Hydrolysegeschwindigkeit hängt insbesondere von den Silicium-ständigen Alkoxygruppen ab, wobei die Methoxygruppe am schnellsten hydrolysiert und mit steigender Kettenlänge oder zunehmender Verzweigung eine Verlangsamung eintritt.

Hydrolyse und Polykondensation können durch den Zusatz von organischen oder anorganischen Basen, wie z. B. Ammoniak oder Aminen, oder organischen oder anorganischen Säuren, wie z. B. Salzsäure oder Ameisensäure, oder aber von üblichen Kondensationskatalysatoren, wie z. B. Dibutylzinndiacetat, beschleunigt werden.

Um ein unterschiedliches Hydrolyse- und Polykondensationsver halten der monomeren Komponenten eines statistischen Copolykondensates auszugleichen, können nach einer Herstellvariante die monomeren Komponenten nach Formel (V), (VI), (VII) und (VIII) vorkondensiert werden. Hierzu werden diese monomeren Komponenten ohne oder unter Verwendung eines die Ausgangsstoffe lösenden Lösungsmittels, bevorzugt eines zu den Alkoxygruppen korrespondierenden linearen oder verzweigten Alkohols mit 1 bis 5 C-Atomen, in Gegenwart einer nicht zur vollständigen Hydrolyse ausreichenden Menge Wasser, vorzugsweise 1 bis 100 Mol% der hierzu benötigten Menge, über einen Zeitraum von 5 Minuten bis zu 5 Tagen bei Raumtemperatur bis 200° C vorkondensiert.

Um diesen Vorkondensationseffekt zu begünstigen, kann dabei ein saurer oder basischer Kondensationskatalysator zugesetzt werden. Bevorzugte Katalysatoren sind z. B. Salpetersäure, Phosphorsäure, Salzsäure, Essigsäure, Ammoniak, Amine, Natronlauge oder Kalilauge oder auch ein metallhaltiger Katalysator, z. B. Di-butylzinndiacetat, die gasförmig oder gelöst in Wasser oder einem organischen Lösungsmittel eingesetzt werden.

Nach erfolgter Vorkondensation wird die vollständige Hydrolyse und Polykondensation, gegebenenfalls nach Zusatz weiteren Wassers und gegebenenfalls nach Zusatz weiteren Lösungsmittels, wie beschrieben, durchgeführt.

Nach einem anderen Verfahren werden sog. Block-Copolykondensate erhalten, bei denen eine Bildung von Blöcken gleicher Einheiten nach Formel (I), (II), (III) und (IV) vorliegt. Dieses Verfahren siehtvor, daß man die monomeren Komponenten nach Formel (V), (VI), (VII) und (VIII) jeweils unabhängig voneinander, ohne oder unter Verwendung eines die Ausgangsstoffe lösenden Lösungsmittels, bevorzugt eines zu den Alkoxygruppen korrespondierenden linearen order verzweigten Alkohols mit 1 bis 5 C-Atomen, in Gegenwart einer nicht zur vollständigen Hydrolyse ausreichenden Menge Wasser, vorzugsweise von 1 bis 100 Mol% der hierzu benötigten Menge, über einen Zeitraum von 5 Minuten bis zu 5 Tagen bei Raumtemperatur bis 200° C, die erhaltenen Kondensate vereinigt und dann gegebenenfalls nach Zusatz weiteren Wassers und gegebenenfalls nach Zusatz weiteren Lösungsmittels die vollständige Hydrolyse

und Polykondensation, wie beschrieben, durchführt

Natürlich kann auch bei dieser Herstellungsvariante einer dervorstehend genannten Kondensationskatalysatoren verwendet werden.

Nach einerweiteren Methode werden sog. gemischte Copolykondensate erhalten, bei denen teilweise eine Bildung von Blöcken gleicher Einheiten nach Formel (I), (II), (III) und (IV) vorliegt, bei denen stets jedoch mindestens eine monomere Komponente nicht vorkondensiert und mindestens eine monomere Komponente vorkondensiert wird.

Dieses Verfahren sieht vor, daß man von den monomeren Komponenten nach Formel (V), (VI), (VII) und (VIII) mindestens ein Monomer aber maximal höchstens 3 Monomere voneinander unabhängig, ohne oder unter Verwendung eines die Ausgangsstoffe lösenden Lösungsmittels, bevorzugtsind zu den Alkoxygruppen korrespondierende lineare oder verzweigte Alkohole mit 1 bis 5 C-Atomen, in Gegenwart einer nicht zur vollständigen Hydrolyse ausreichenden Menge Wasser, vorzugsweise von 1 bis 100 Mol% der hierzu benötigten Menge, über einen Zeitraum von 5 Minuten bis zu 5 Tagen bei Raumtemperatur bis 200° C vorkondensiert, das erhaltene Vorkondensat bzw. die erhaltenen Vorkondensate und mindestens eine nichtvorkondensierte Komponente miteinander vereinigt und dann gegebenenfalls nach Zusatz weiteren Wassers und gegebenenfalls nach Zusatz weiteren Lösungsmittels die vollständige Hydrolyse und Polykondensation, wie beschrieben, durchführt.

Die Verwendung eines sauren, basischen und/oder metallhaltigen Kondensationskatalysators zur Vorkondensation ist auch bei dieser Herstellungsvariante möglich und die weitere Behandlung des gebildeten Polykondensates gestaltet sich ebenso wie bei den anderen beschriebenen Herstellungsverfahren auch.

Bei der Durchführung einer Vorkondensation hängt die verwendete Menge Wasserdavon ab, welcher Oligomerisierungsgrad, d. h. welche Blockgröße erreicht werden soll. Bei Einsatz von mehr Wasser zur Vorkondensation und/oder längerer Vorkondensationszeit entstehen natürlich grundsätzlich größere Einheiten als bei Verwendung von weniger Wasser und/oder einer kürzeren Reaktionszeit.

Die Dauer der Vorkondensation hängt, wie bereits vorstehend beschrieben, generell auch von der Hydrolysebereitschaft der monomeren Komponenten und der Temperatur ab.

Charakterisiert sind die Füllstoffe für die neuen Dentalmaterialien insbesondere anhand der quantitativen Hydrolyse- und Kondensationsausbeute und der Elementaranalysen. Zwischen den nach den unterschiedlichen Herstellungsverfahren erhaltenen Copolykondensaten besteht rein optisch kein Unterschied. Je nach Behandlung besitzen die erfindungsgemäßen Füllstoffe Oberflachen von ca. 0 bis 200 $m^2/g$. Die gewünschten Teilchengrößendurchmesser von 0,01 μm bis 100 μm lassen sich problemlos durch Anwendung gängiger Mahltechniken einstellen.

Ein weiterer Gegenstand der Erfindung ist die Verwendung des Dentalmaterials zur Fertigung von, Inlays, Verblendungen, Kronen, Brücken, Zahnprothesen, künstlichen Zähnen und Klebern zur Befestigung von Inlays, Kronen und Brücken.

Die Erfindung wird nachfolgend anhand von Ausführungsbeispielen weiter erläutert.

## I. Herstellung dar erfindungsgemäßen Füllstoffe

Beispiel 1

1404,2 g (6,74 Mol) $Si(OC_2H_5)_4$ und 95,7 g (0,337 Mol) $Ti(i\text{-}OC_3H_7)_4$ wurden in 200 ml 1n ethanolischer HCl-Lösung vereinigt und zunächst in einem 3 l-Dreihalskolben mit KPG-Rührerund Rückflußkühler über einen Zeitraum von 5 Stunden bei Rückflußtemperatur vorkondensiert. Nach dieser Zeit wurde die Lösung zunächst mit 300 ml Ethanol verdünnt und dann binnen 10 Minuten mit 525 ml 10 %iger Ammoniaklösung versetzt. Es wurde weitere 2 Stunden unter Rückfluß gerührt, dann der Ansatz abgekühlt und der gebildete Feststoff über eine Saugnutsche abfiltriert sowie mit 2 x 500 ml Wasser gewaschen. Der er haltene Feststoffwurde anschließend in einen 3 l-Autoklaven überführt, mit 1 l 5 %iger Ammoniaklösung versetzt und 24 Stunden bei 150° C gerührt. Nach erneuter Abfiltration, Waschen mit 1 l Wasser und 15-stündiger Trocknung bei 120° C unter $N_2$-Atmosphäre wurden 429,0 g (99,3 % der Theorie) eines Dentalfüllstoffs, bestehend aus Polymereinheiten der Formel

$$TiO_2 \cdot 20\ SiO_2$$

erhalten. Das Produkt wurde anschließend 24 Stunden in einer Kugelmühle gemahlen.

Diese Aufgabe wird durch die Bereitstellung eines neuen pastösen, in Gegenwart eines Initiators zu einer auf Hochglanz polierbaren Masse aushärtbaren Dentalmaterials aus einem polymerisierbaren organischen Bindemittel gelöst, enthaltend mindestens ein polymerisierbares Acrylat oder Methacrylat und 20 bis 90 Gew.-%, bezogen auf das Gesamtgewicht, eines feinteiligen Füllstoffs aus einem Heterosiloxan, welches eine spezifische Oberfläche von ca. 0 bis 200 $m^2/g$ und eine Partikelgröße von 0,01 bis 100 μm aufweist und aus Einheiten der Formeln

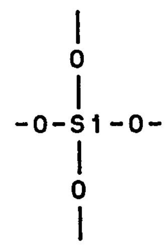

(I)

und

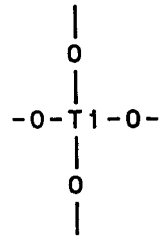

(II)

und

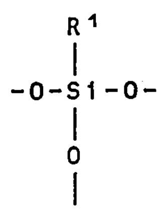

(III)

wobei $R^1$ für eine lineare oder verzweigte Alkylgruppe mit 1 bis 18 C-Atomen, eine Cycloalkylgruppe mit 5 bis 8 C-Atomen, eine Phenylgruppe oder eine Arylalkylgruppe oder eine mit einem Acrylat- oder Methacrylatrest verbundene lineare, gegebenenfalls verzweigte Alkylgruppe mit 1 bis 6 C-Atomen oder für einen einfach olefinisch ungesättigten, vorzugsweise endständig ungesättigten linearen, gegebenenfalls verzweigten Kohlenwasserstoffrest mit 2 bis 8 C-Atomen oder für einen zyklischen, einfach olefinisch ungesättigten Kohlenwasserstoffrest mit 5 - 8 C-Atomen steht, und gegebenenfalls Einheiten der Formel

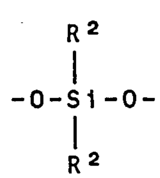

(IV)

aufgebaut ist, in der $R^2$ eine Methyl-, Ethyl- oder Phenylgruppe repräsentiert und die freien Valenzen der an die Silicium- bzw. Titanatome gebundenen Sauerstoffatome bei den Einheiten (I) bis (IV) wie bei Kieselsäuregerüsten durch ein Siliciumatom einer gleichen oder einer unterschiedlichen Einheit und/oder durch ein Titanatom abgesättigt sind, wobei das Verhältnis der Summe der Siliciumatome aus den Einheiten der Formel (I) , (III) und (IV) zu den Titanatomen aus den Einheiten der Formel (II) 1 : 1 bis 100 : 1 und der Anteil der Siliciumatome aus den Einheiten (I) zu der Gesamtmenge der Siliciumatome 100 Mol% bis 30 Mol% beträgt und das erhältlich ist

A) in Gestalt eines statistischen Copolykondensats, indem man

a) ein Alkoxysilan der allgemeinen Formel

$$Si(OR^3)_4 \quad (V)$$

wobei $R^3$ für eine lineare oder verzweigte Alkylgruppe mit 1 bis 5 C-Atomen steht, und eine Titanverbindung der allgemeinen Formel

$$Ti(OR^3)_4 \quad (VI)$$

in der $R^3$ dieselbe Bedeutung wie in Formel (V) hat und ein Alkoxysilan der allgemeinen Formel

$$R^1\text{-}Si(OR^3)_3 \quad (VII)$$

in der $R^1$ wie unter Formel (III) und $R^3$ wie unter Formel (V) definiert sind und/oder ein Alkoxysilan der allgemeinen Formel

$$R^2_2\ Si(OR^3)_2 \quad (VIII)$$

in der $R^2$ wie unter Formel (IV) und $R^3$ wie unter Formel (V) definiert sind, in einem weitgehend wassermischbaren, aber die Verbindungen nach Formeln (V) bis (VIII) lösenden Lösungsmittel auflöst und der Lösung unter Rühren eine zumindest für die vollständige Hydrolyse und Kondensation ausreichende Menge Wasser zufügt und das Reaktionsgemisch unter Weiterrühren bei einer bestimmten Temperatur im Bereich von Raumtemperatur bis 200° C vorkondensiert,

b) den sich bildenden Feststoff gegebenenfalls nach Zusatz weiteren Lösungsmittels oder Wassers noch 1 Stunde bis zu 6 Stunden bei 60° bis 200° C, bei Normaldruck oder einem Druck rührt, welcher der Summe der Partialdrucke bei der jeweiligen Temperatur entspricht,

c) das als Feststoff gebildete Heterosiloxan nach gängigen Techniken von der flüssigen Phase abtrennt, gegebenenfalls wäscht,

d) in Wasser, einem Wasser/Alkohol-Gemisch oder in reinem Alkohol, in Gegenwart eines sauren oder eines basischen Katalysators, nachkondensiert und

e) gegebenenfalls bei Raumtemperatur bis 200° C, gegebenenfalls unter Schutzgasatmosphäre oder im Vakuum trocknet, gegebenenfalls 1 bis 100 Stunden bei Temperaturen von 150° C bis 300° C unter Schutzgasatmosphäre oder im Vakuum tempert und gegebenenfalls mahlt und/oder klassifiziert.

B) in Gestalt eines Block-Copolykondensats, indem man

a) die monomeren Komponenten nach Formel (V), (VI) , (VII) und (VIII) jeweils unabhängig voneinander, ohne oder unter Einsatz eines die Ausgangsstoffe lösenden Lösungsmittels, in Gegenwart einer nicht zur vollständigen Hydrolyse ausreichenden Menge Wasser, über einen Zeitraum von 5 Minuten bis zu 5 Tagen bei Raumtemperatur bis 200° C vorkondensiert, die erhaltenen Vorkondensate vereinigt und nach Zusatz weiteren Wassers und/oder weiteren Lösungsmittels die unter A) genannten Schritte b) bis e) anschließt oder

C) in Gestalt eines gemischten Copolykondensats, indem man

a) von den monomeren Komponenten nach Formel (V), (VI), (VII) und (VIII) mindestens ein Monomer aber maximal höchstens 3 Monomere voneinander unabhängig, wie bei B) vorkondensiert, das erhaltene Vorkondensat oder die erhaltenen Vorkondensate und mindestens eine nicht vorkondensierte Komponente miteinander vereinigt und die unter A) genannten Schritte b) bis e) anschließt,

**dadurch gekennzeichnet,**

daß man aie Nachkondensation im Autoklaven über einen Zeitraum von 1 Stunde bis zu 5 Tagen bei Temperaturen von 60° C bis 250° C unter einem Druck, welcher der Summe der Partialdrucke bei der jeweiligen Temperatur entspricht, durchführt.

Die Füllstoffe werden in den Dentalmassen in einer Menge von 20 bis 90 Gew.%, vorzugsweise 40 bis 90 Gew.%, eingesetzt.

Die erfindungsgemäßen Füllstoffe müssen, sofern keine Doppelbindungsfunktionalität gegeben ist, natürlich vor ihrer Einbringung in die organische Polymermatrix mit einer geeigneten Organosilanverbindung, vorzugsweise 3-Methacryloyloxypropyltrimethoxy- oder 3-Methacryloyloxypropyltriethoxysilan, umgesetzt werden. Dies schließt jedoch nicht aus, daß auch Doppelbindungs-funktionelle Füllstoffe noch zusätzlich silanisiert werden.

Analoges gilt auch für eine weitere, aus Gründen der besonders leichten Verfügbarkeit der Ausgangsmaterialien vorteilhafte erfindungsgemäße Füllstoffzusammensetzung, die einen Aufbau des Heterosiloxans aus Einheiten der Formel (I), (II) und den speziellen Einheiten der Formel (IV)

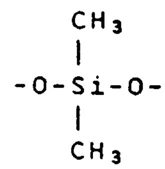

vorsieht, wobei das molare Verhältnis der Summe der Einheiten der Formel (I) und (IV) zu den Einheiten der Formel (II) 1 : 1 bis 100 : 1 betragt.

| Analysen: | % Ti | % Si |
|---|---|---|
| Theorie: | 3,74 | 43,83 |
| Gefunden: | 3,70 | 43,40 |

Spezifische Oberfläche: 72 $m^2/g$

Beispiel 2

1426,0 g (6,845 Mol) $Si(OC_2H_5)_4$ und 48,6 g (0,171 Mol) $Ti(OC_3H_7)_4$ wurden in 500 ml Isopropanol, die 1 normal an HCl war, vereinigt. Die Lösung wurde mit 1 ml $H_2O$ versetzt und dann 24 Stunden unter Rückfluß gerührt. Nach dieser Zeit wird das Vorkondensat mit 25,4 g (0,171 Mol) $(CH_3)_2Si(OC_2H_5)_2$ und mit 400 ml Wasser versetzt. Nach der Feststoffbildung wurden 750 ml Wasser zur Verdünnung zugesetzt und noch 2 Stunden unter Rückfluß gerührt. Dann wurde abgekühlt, der gebildete Feststoff abfiltriert und in einer 5 %igen Ammoniaklösung suspendiert. Die Suspension wurde in einen Autoklaven überführt und dann 48 Stunden bei 130° C unter Eigendruck gerührt. Nach dem Abkühlen, Abfiltrieren des Feststoffs, Waschen mit 2 l Wasser, 10-stündigem Trocknen bei 120° C und 12-stündigem Tempern bei 160° C unter $N_2$-Atmosphäre wurden 434,0 g (99,2 % der Theorie) eines Dentalfüllstoffs, bestehend aus Einheiten der Formel

$$TiO_2 \cdot (CH_3)_2SiO_{2/2} \cdot 40SiO_2$$

erhalten. Das Produkt wurde anschließend 24 Stunden in einer Kugelmühle gemahlen.

| Analysen: | % C | % H | % Si | % Ti |
|---|---|---|---|---|
| Theorie: | 0,94 | 0,24 | 45,03 | 1,87 |
| Gefunden: | 0,96 | 0,20 | 44,84 | 1,80 |

Spezifische Oberfläche: 37 $m^2/g$

## Beispiel 3

711,7 g (3,416 Mol) $Si(OC_2H_5)_4$, 14,0 g (0,085 Mol) $n\text{-}C_3H_7\text{-}Si(OCH_3)_3$ und 24,3 g (0,085 Mol) $Ti(OC_3H_7)_4$ wurden in 250 ml 1n ethanolischer HCl-Lösung vereinigt und zunächst 4 Stunden unter Rückfluß gerührt. Nach dieser Zeit wurden weitere 150 ml Ethanol sowie 250 ml entsalztes Wasser zugegeben und nach einer halben Stunde weiteren Rührens unter Rückfluß nochmals 375 ml entsalztes Wasser zur Verdünnung. Dann wurde noch weitere 2 Stunden unter Rückfluß gerührt und anschließend abgekühlt, der Feststoff abfiltriert mit 2 l Wasser gewaschen und in einer 10 %igen wäßrigen Ammoniaklösung eingeschlämmt. Nach 24-stündiger Temperaturbehandlung der Suspension bei 150° C wurde analog zu Beispiel 1 aufgearbeitet und gemahlen. Erhalten wurden 214,3 g (97,3 % der Theorie) eines Dentalfüllstoffes, bestehend aus Polymereinheiten der Formel

$$TiO_2 \cdot n\text{-}C_3H_7\text{-}SiO_{3/2} \cdot 40Sio_2$$

| Analysen: | % C | % H | % Si | % Ti |
|---|---|---|---|---|
| Theorie: | 1,40 | 0,27 | 44,66 | 1,86 |
| Gefunden: | 1,50 | 0,31 | 44,35 | 1,77 |

Spezifische Oberfläche: 48 $m^2/g$

## Beispiel 4

704,9 g (3,384 Mol) $Si(OC_2H_5)_4$, 21,0 g (0,085 Mol)

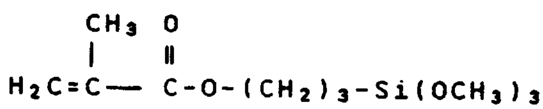

und 24,1 g (0,085 Mol) $Ti(OC_3H_7)_4$ wurden in 250 ml 1n ethanolischer HCl-Lösung 2 Stunden lang bei Rückflußtemparatur vorkondensiert Anschließend wurden weitere 125 ml Ethanol und 250 ml 10 %iges Ammoniakwasser zugesetzt. Nach einer halben Stunde Rühren unter Rückfluß wurde mit 350 ml Wasser verdünnt. Nach weiterer Verfahrensweise analog zu Beispiel 1 wurden 220,6g (97,9% der Theorie) eines Dentalfüllstoffs bestehend aus Einheiten der Formel

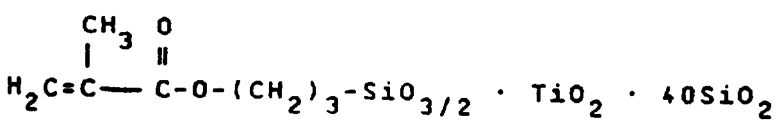

erhalten.

| Analysen: | % C | % H | % Si | % Ti |
|---|---|---|---|---|
| Theorie: | 3,16 | 0,42 | 43,25 | 1,80 |
| Gefunden: | 3,07 | 0,37 | 42,76 | 1,75 |

Spezifische Oberfläche: 30 $m^2/g$

Beispiel 5

727,8 g (3,49 Mol) $Si(OC_2H_5)_4$ wurden mit 2 ml 1 %iger wäßriger Ammoniaklösung versetzt und 4 Stunden bei 100° C gerührt. Gleichzeitig wurden 19,0 g (0,07 Mol) $(C_6H_5)_2Si(OC_2H_5)_2$ mit 2 ml 1 normaler ethanolischer HCl-Lösung versetzt und ebenfalls 4 Stunden bei 100° C gerührt. Analog wurden 23,7 g (0,07 Mol) $Ti(OC_4H_9)_4$ nach Zusatz von 2 ml 1 normaler ethanolischer HCl-Lösung ebenfalls 4 Stunden bei 100° C gerührt. Nach dieser Zeit wurden die 3 Vorkondensate vereinigt, mit 300 ml Ethanol sowie 150 ml $H_2O$ versetzt und unter Rückfluß gerührt, bis die Gelierung einsetzte. Es wurde mit 200 ml Ethanol verdünnt, noch 2 Stunden unter Rückfluß gerührt, dann abgefüllt, der Feststoff abfiltriert, mit 2 l Wasser gewaschen und in einer 1n HCl-Lösung eingeschlämmt. Nach 24-stündiger Nachbehandlung bei 130° C im Autoklaven wurde analog zu Beispiel 1 aufgearbeitet. Erhalten wurden 223,2 g (97,5 % der Theorie) eines Dentalfüllstoffes, bestehend aus Einheiten der Formel

$$TiO_2 \cdot (C_6H_5)_2SiO_{2/2} \cdot 50SiO_2$$

| Analysen: | % C | % H | % Si | % Ti |
|---|---|---|---|---|
| Theorie: | 4,39 | 0,31 | 43,64 | 1,46 |
| Gefunden: | 4,16 | 0,25 | 42,91 | 1,31 |

Spezifische Oberfläche: 86 $m^2/g$

Beispiel 6

727,8 g (3,49 Mol) $Si(OC_2H_5)_4$, 22,2g (0,1165 Mol) $CH_2$=CH-$Si(OC_2H_5)_3$ und 33,1 g (0,1165 Mol) $Ti(OC_3H_7)_4$ wurden miteinander vereinigt. Die Mischung wurde mit 3 ml 1n Essigsäurelösung versetzt und 12 Stunden bei 80° C gerührt. Nach dieser Zeit wurde die Mischung mit 400 ml Ethanol sowie 200 ml Wasser versetzt und weitere 3 Stunden unter Rückfluß gerührt. Nach weiterer Verfahrensweise analog zu Beispiel 1 wurden 221,2 g (96,8 % der Theorie) eines Dentalfüllstoffes, bestehend aus Einheiten der Formel

$$H_2C{=}CH\text{-}SiO_{3/2} \cdot TiO_2 \cdot 30SiO_2$$

erhalten.

| Analysen: | % C | % H | % Si | % Ti |
|---|---|---|---|---|
| Theorie: | 1,22 | 0,15 | 44,39 | 2,44 |
| Gefunden: | 1,10 | 0,10 | 43,97 | 2,30 |

Spezifische Oberfläche: 62 $m^2/g$

**II. Herstellung der erfindungsgemäßen Dentalwerkstoffe**

Zur Herstellung der erfindungsgemäßen Dentalmassen wurden die Füllstoffe aus den Beispielen Nr.1 bis Nr.4 mit einer Kugelmühle auf mittlere Korngrößen zwischen ca. 3 bis 7 µm heruntergemahlen. Dann wurden die Füllstoffe mit 3-Methacryloyloxypropyltrimethoxysilan nach üblichen Verfahren silanisiert.

Die Füllstoffe wurden in Mengen von ca. 58 bis 67 % (m/m) in eine Monomermatrix eingebracht, wie sie für Dentalkunststoffe üblicherweise verwendet wird. Es wurden noch Initiatoren zugesetzt, und die Massen wurden zu homogenen Pasten geknetet.

Es wurde eine Reihe von physikalischen Eigenschaften an aus den verschiedenen Pasten hergestellten ausgehärteten Prüfkörpern bestimmt und mit denen von Handelsprodukten und Laborvergleichsprodukten verglichen (Tabelle I).

Beispiele für erfindungsgemäße Dentalmassen

1. Heißhärtende erfindungsgemäße Dentalmassen

Die Herstellung der Prüfkörper aus den heißhärtenden erfindungsgemäßen Dentalmassen erfolgte in der Weise, daß die Massen in die entsprechende Prüfkörperformen gepreßt und dann in einem Wasserbad unter 6 atü Druck bei 90° C 30 Minuten lang ausgehärtet wurden.

Beispiel Nr. 1 (Angaben in Gewichtsteilen):

| | |
|---|---|
| 61,0 | Füllstoff Nr. 1 |
| 26,7 | Bis-GMA |
| 11,7 | TEDMA |
| 0,4 | Dibenzoylperoxid |

Beispiel Nr. 2 (Angaben in Gewichtsteilen):

| | |
|---|---|
| 58,0 | Füllstoff Nr. 2 |
| 29,0 | Bis-GMA |
| 12,6 | TEDMA |
| 0,4 | Dibenzoylperoxid |

Beispiel Nr. 3 (Angaben in Gewichtsteilen):

| | |
|---|---|
| 67,0 | Füllstoff Nr. 3 |
| 23,8 | Bis-GMA |
| 9,9 | TEDMA |
| 0,3 | Dibenzoylperoxid |

Beispiel Nr. 4 (Angaben in Gewichtsteilen):

| | |
|---|---|
| 67,0 | Füllstoff Nr. 4 |
| 23,8 | Bis-GMA |
| 9,9 | TEDMA |
| 0,3 | Dibenzoylperoxid |

2. Lichthärtende erfindungsgemäße Dentalmasse

Die lichthärtende erfindungsgemäße Dentalmasse bestehtaus einer weißen Paste, die durch Bestrahlung mit einer zahnärztlichen Halogenlichtlampe (Translux, Fa. Kulzer) ausgehärtet wird. Bestrahlungszeit 100 Sek.

Beispiel Nr. 5 (Angaben in Gewichtsteilen):

| | |
|---|---|
| 61,0 | Füllstoff Nr. 2 |
| 26,4 | Bis-GMA |
| 11,6 | TEDMA |
| 0,2 | Campherchinon |
| 0,1 | N,N-Dimethyl-p-toluidin |

Abkürzungen:

| | |
|---|---|
| Bis-GMA: | 2,2-Bis[p-(γ-methacrylolyoxy-β-hydroxypropoxy)-phenyl]-propan |
| TEDMA: | Triethylenglykoldimethacrylat |

Handelsprodukte, mit denen die erfindungsgemäßen Dentalmassen verglichen werden:

Handelsprodukte

Konventionelle Composite (Estilux, Fa. Kulzer): Als Füllstoff dient ein silanisiertes Lithiumaluminiumglas einer mittleren Korngröße von ca. 4 µm. Der Füllstoffgehalt liegt bei ca. 75 % (m/m).

Hybrid-Composite (Degufill H, Degussa): Als Füllstoff dienen silanisiertes Bariumaluminiumsilikatglas einer mittleren Korngröße von ca. 2. µm, welches aber zu 100 % feiner als 5 µm ist, sowie silanisiertes, hochdisperses $SiO_2$. Der Füllgrad an Glas beträgt ca. 70% (m/m), der an hochdispersem $SiO_2$ ca. 11% (m/m). Daraus ergibt sich ein Gesamtgehalt an anorganischen Füllstoffen von ca. 8 % (m/m).

Microfüller Composite (Durafill, Fa. Kulzer): Als Füllstoff dient silanisiertes, hochdisperses $SiO_2$ einer mittleren Korngröße zwischen 0,01 bis 0,04 µm. Der Füllgrad beträgt ca. 50 % (m/m).

Die Aushärtung aller Massen erfolgte mit dem Lichtgerät Translux (Fa. Kulzer) und einer Bestrahlungszeit von 40 Sek.

Lichthärtende Laborversuche = VP

(Angaben in Gewichtsteilen):

VP1:

| | |
|---|---|
| 75 | Bariumaluminiumsilikatglas, silanisiert (mittlere Korngröße ca. 4 µm) |
| 17 | Bis-GMA |
| 7,8 | TEDMA |
| 0,2 | Dibenzoylperoxid |

VP2:

| | |
|---|---|
| 35 | Bis-GMA |
| 14,5 | TEDMA |
| 50 | hochdisperses $SiO_2$, silanisiert (mittlere Korngröße 0,01 - 0,04 µm) |
| 0,5 | Dibenzoylperoxid |

Die Aushärtung dieser Pasten erfolgtanalog zur Aushärtung der heißhärtenden erfindungsgemäßen Massen.

Prüfung und Beurteilung der Polierbarkeit

Von allen Materialien wurden Prüfkörper eines Durchmessers von 15 mm und einer Dicke von 3 mm hergestellt. Die Oberflächen aller Prüfkörper wurden zunächst mit feinem Schleifpapier (600 grit) gleichmäßig beschliffen. Dann wurden sie unter Wasser mit feinstteiligern Aluminiumoxid (mittlere Teilchengröße 0,04 µm) auf einem Baumwolltuch poliert.

Die Polierbarkeit wurde visuell beurteilt und mittels einer Punkteskala zwischen 1 bis 5 benotet, wobei 1 = matt und 5 = hochglänzend ist.

Tabelle I: Eigenschaften der erfindungsgemäßen Dentalmassen im Vergleich zu Handelsprodukten sowie zu konventionellen und microgefüllten heißhärtenden Dentalmassen

| Eigenschaft | Nr. 1 | Nr.2 | Nr. 3 | Nr.4 | Nr.5 | Estilux | Durafill | Degufill H | VP1 | VP2 |
|---|---|---|---|---|---|---|---|---|---|---|
| Biegefestigkeit [$N/mm^2$] DIN 13 | 100 | 74 | 85 | 107 | 128 | 100 | 62 | 110 | 140 | 65 |
| Biegemodul [$N/mm^2$] DIN | 6000 | 6000 | 6800 | 6600 | 6800 | 9700 | 3200 | 8000 | 11000 | 3900 |
| Druckfestigkeit [$N/mm^2$] | 455 | 470 | 381 | 430 | 410 | 300 | 350 | 320 | 350 | 410 |
| Vickers Härte HV 5 [$N/mm^2$] DIN | 620 | 550 | 710 | 880 | 820 | 660 | 290 | 680 | 660 | 330 |
| Polierbarkeit | 5 | 5 | 5 | 5 | 5 | 1 | 4 | 3 | 1 | 4 |

**Patentansprüche**

1. Pastöses, in Gegenwart eines Initiators zu einer auf Hochglanz polierbaren Masse aushärtbares Dentalmaterial aus einem polymerisierbaren organischen Bindemittel enthaltend mindestens ein polymerisierbares Acrylat oder Methacrylat und 20 bis 90 Gew.%, bezogen auf das Gesamtgewicht, eines feinteiligen Füllstoffs aus einem Heterosiloxan, welches eine spezifische Oberfläche von ca 0 bis 200 $m^2/g$ und eine Partikelgröße von 0,01 µm bis 100 µm aufweist und aus Einheiten der Formeln

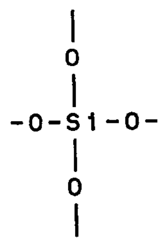

(I)

und

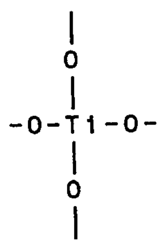

(II)

und

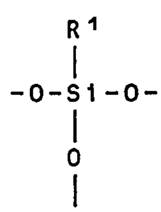

(III)

wobei $R^1$ für eine lineare oder verzweigte Alkylgruppe mit 1 bis 18 C-Atomen, eine Cycloalkylgruppe mit 5 bis 8 C-Atomen, eine Phenylgruppe oder eine Arylalkylgruppe oder eine mit einem Acrylat- oder Methacrylatrest verbundene lineare, gegebenenfalls verzweigte Alkylgruppe mit 1 bis 6 C-Atomen oder für einen einfach olefinisch ungesättigten, vorzugsweise endständig ungesättigten linearen, gegebenenfalls verzweigten Kohlenwasserstoffrest mit 2 bis 8 C-Atomen oder für einen zyklischen, einfach olefinisch ungesättigten Kohlenwasserstoffrest mit 5 - 8 C-Atomen steht, und gegebenenfalls Einheiten der Formel

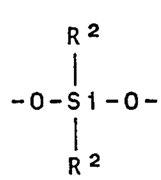

(IV)

aufgebaut ist, in der $R^2$ eine Methyl-, Ethyl- oder Phenylgruppe repräsentiert und die freien Valenzen der an die Silicium- bzw. Titanatome gebundenen Sauerstoffatome bei den Einheiten (I) bis (IV) wie bei Kieselsäuregerüsten durch ein Siliciumatom einer gleichen oder einer unterschiedlichen Einheit und/oder durch ein Titanatom abgesättigt sind, wobei das Verhältnis der Summe der Siliciumatome aus den Einheiten der Formel (I), (III) und (IV) zu den Titanatomen aus den Einheiten der Formel (II) 1 : 1 bis 100 : 1 und der Anteil der Siliciumatome aus den Einheiten (I) zu der Gesamtmenge der Siliciumatome 100 Mol% bis 30 Mol% beträgt und das erhältlich ist

A) in Gestalt eines statistischen Copolykondensats, indem man

a) ein Alkoxysilan der allgemeinen Formel

$$Si(OR^3)_4 \qquad (V)$$

wobei $R^3$ für eine lineare oder verzweigte Alkylgruppe mit 1 bis 5 C-Atomen steht, und eine Titanverbindung der allgemeinen Formel

$$Ti(OR^3)_4 \qquad (VI)$$

In der $R^3$ dieselbe Bedeutung wie in Formel (V) hat und ein Alkoxysilan der allgemeinen Formel

$$R^1 - Si(OR^3)_3 \qquad (VII)$$

in der $R^1$ wie unter Formel (III) und $R^3$ wie unter Formel (V) definiert sind und/oder ein Alkoxysilan der allgemeinen Formel

$$R^2_2\, Si(OR^3)_2 \qquad (VIII)$$

in der $R^2$ wie unter Formel (IV) und $R^3$ wie unter Formel (V) definiert sind, in einem weitgehend wassermischbaren, aber die Verbindungen nach Formeln (V) bis (VIII) lösenden Lösungsmittel auflöst und der Lösung unter Rühren eine zumindest für die vollständige Hydrolyse und Kondensation ausreichende Menge Wasser zufügt und das Reaktionsgemisch unter Weiterrühren bei einer bestimmten Temperatur im Bereich von Raumtemperatur bis 200° C vorkondensiert,

b) den sich bildenden Feststoff gegebenenfalls nach Zusatz weiteren Lösungsmittels oder Wassers noch 1 Stunde bis zu 6 Stunden bei 60° bis 200° C, bei Normaldruck oder einem Druck rührt, welcher der Summe der Partialdrucke bei der jeweiligen Temperatur entspricht,

c) das als Feststoff gebildete Heterosiloxan nach gängigen Techniken von der flüssigen Phase abtrennt, gegebenenfalls wäscht,

d) in Wasser, einem Wasser/Alkohol-Gemisch oder in reinem Alkohol, in Gegenwart eines sauren oder eines basischen Katalysators, nachkondensiert und

e) gegebenenfalls bei Raumtemperatur bis 200° C, gegebenenfalls unter Schutzgasatmosphäre oder im Vakuum trocknet, gegebenenfalls 1 bis 100 Stunden bei Temperaturen von 150° C bis 300° C unter Schutzgasatmosphäre oder im Vakuum tempert und gegebenenfalls mahlt und/oder klassifiziert.

B) in Gestalt eines Block-Copolykondensats, indem man

a) die monomeren Komponenten nach Formel (V), (VI), (VII) und (VIII) jeweils unabhängig voneinander, ohne oder unter Einsatz eines die Ausgangsstoffe lösenden Lösungsmittels, in Gegenwart einer nicht zur vollständigen Hydrolyse ausreichenden Menge Wasser, über einen Zeitraum von 5 Minuten bis zu 5 Tagen bei Raumtemperatur bis 200° C vorkondensiert, die erhaltenen Vorkondensate vereinigt und nach Zusatz weiteren Wassers und/oder weiteren Lösungsmittels die unter A) genannten Schritte b) bis e) anschließt oder

C) in Gestalt eines gemischten Copolykondensats, indem man

a) von den monomeren Komponenten nach Formel (V), (VI), (VII) und (VIII) mindestens ein Monomer aber maximal höchstens 3 Monomere voneinander unabhängig, wie bei B) vorkondensiert, das erhaltene Vorkondensat oder die erhaltenen Vorkondensate und mindestens eine nicht vorkondensierte Komponente miteinander vereinigt und die unter A) genannten Schritte b) bis e) anschließt,

**dadurch gekennzeichnet,**
daß man die Nachkondensation im Autoklaven über einen Zeitraum von 1 Stunde bis zu 5 Tagen bei Temperaturen von 60° C bis 250° C unter einem Druck, welcher der Summe der Partialdrucke bei der jeweiligen Temperatur entspricht, durchführt.

**2.** Dentalmaterial nach Anspruch 1,
**dadurch gekennzeichnet,**
daß der feinteilige Füllstoff aus dem Heterosiloxan in einer Menge von 40 bis 90 Gew.-%, bezogen auf das Gesamtgewicht, vorliegt.

**3.** Dentalmaterial nach Anspruch 1,
**dadurch gekennzeichnet,**
daß der feinteilige Füllstoff aus dem Heterosiloxan eine spezifische Oberfläche ca 0 bis 100 $m^2/g$ und eine Partikelgröße von 0,1 µm bis 30 µm aufweist.

**4.** Dentalmaterial gemäß Anspruch 1,
**dadurch gekennzeichnet,**
daß es als Füllstoff ein Heterosiloxan enthält, das aus Einheiten der Formel (I), (II) und den speziellen Einheiten der Formel (IV)

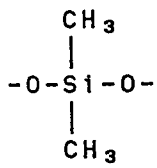

besteht, wobei das molare Verhältnis der Summe der Einheiten der Formel (I) und (IV) zu den Einheiten der Formel (II) 1 : 1 bis 100 : 1 beträgt.

**5.** Dentalmaterial gemäß Anspruch 1,
**dadurch gekennzeichnet,**
daß sein Füllstoff in Gestalt eines statistischen Copolykondensats erhältlich ist, indem man die monomeren Komponenten nach Formel (V), (VI), (VII) und (VIII) ohne oder unter Einsatz eines die Ausgangsstoffe lösenden Lösungsmittels, bevorzugt eines zu den Alkoxygruppen korrespondierenden linearen oder verzweigten Alkohols mit 1 bis 5 C-Atomen, in Gegenwart einer nicht zur vollständigen Hydrolyse ausreichenden Menge Wasser, vorzugsweise von 1 bis 100 Mol% der hierzu benötigten Menge, über einen Zeitraum von 5 Minuten bis zu 5 Tagen bei Raumtemperatur bis 200° C vorkondensiert.

**6.** Dentalmaterial nach Anspruch 1,
**dadurch gekennzeichnet,**
daß man bei der Herstellung des Block-Copolykondensats und des gemischten Copolykondensats die Ausgangsstoffe in einem zu deren Alkoxygruppen korrespondierenden, linearen oder verzweigten Alkohol mit 1 bis 5 C-Atomen auflöst.

**7.** Dentalmaterial gemäß den Ansprüchen 1 - 3,
**dadurch gekennzeichnet,**
daß das statistische Copolymerisat erhältlich ist, indem man Hydrolyse und Kondensation in Methanol, Ethanol , n-und i-Propanol, n- und i-Butanol oder n-Pentanol durchführt.

**8.** Dentalmaterial nach Anspruch 1,
**dadurch gekennzeichnet,**

daß man bei der Herstellung des Block-Copolykondensats im Schritt a) Wasser in einer Menge von 1 bis 100 Mol-% der zur vollständigen Hydrolyse ausreichenden Menge einsetzt.

**9.** Dentalmaterial gemäß den Ansprüchen 1 bis 4,
**dadurch gekennzeichnet,**
daß sein Füllstoff erhältlich ist, indem man zur Vorkondensation einen sauren, basischen und/oder metallhaltigen Kondensationskatalysator einsetzt.

**10.** Dentalmatertal nach Anspruch 1,
**dadurch gekennzeichnet,**
daß man im Schritt als basischen Katalysator für die Nachkondensation Ammoniak einsetzt.

**11.** Verwendung des Dentalmaterials nach vorstehenden Ansprüchen zur Fertigung von, Inlays, Verblendungen, Kronen, Brücken, Zahnprothesen, künstlichen Zähnen und Klebern zur Befestigung von Inlays, Kronen und Brücken.

## Claims

**1.** Paste-like dental material which, in the presence of an initiator, may be cured into a composition polishable to a high gloss, and which is prepared from a polymerisable organic binder, containing at least one polymerisable acrylate or methacrylate and 20 to 90 wt. % of a heterosiloxane filler, related to the sum of weight, having a specific surface area of about 0 to 200 $m^2/g$ and a particle size of 0,01 µm to 100 µm and a which is composed of units of the formula

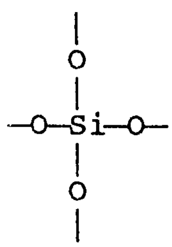

(I)

and units of the formula

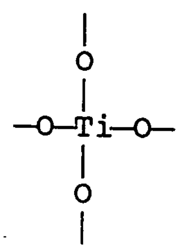

(II)

and units of the formula

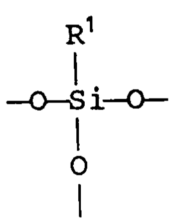

(III)

wherein $R^1$ stands for a linear or branched alkyl group with 1 to 18 C atoms, a cycloalkyl group with 5 to 8 C atoms, a phenyl group or an arylalkyl group or a linear, optionally branched, alkyl group with 1 to 6 C atoms, which alkyl

group is bonded to an acrylate or methacrylate residue, or for a singly olefinically unsaturated, preferably terminally unsaturated, linear, optionally branched, hydrocarbon residue with 2 to 8 C atoms or for a cyclic, singly olefinically unsaturated hydrocarbon residue with 5 to 8 C atoms and/or represents optionally units of the formula

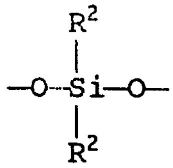

(IV)

wherein $R^2$ stands for a methyl, ethyl- or phenylgroup, and the free valencies of the oxygen atoms bonded to the silicon or titanium atoms in units (I) to (IV) are saturated, as in silicic acid skeletal structures, by a silicon atom of an identical or different unit and/or by a titanium atom, wherein the ratio of the sum of silicon atoms from the units of formulae (I), (III) and (IV) to the titanium atoms from the units of formula (II) is 1:1 to 100:1 and the proportion of silicon atoms from the units (I) to the total quantity of silicon atoms is 100 mol% to 30 mol%,
obtainable

A) in the form of a random

a) copolycondensate may be obtained by dissolving an alkoxysilane of the general formula

$$Si(OR^3)_4 \qquad (V)$$

wherein $R^3$ stands for a linear or branched alkyl group with 1 to 5 C atoms, and a titanium compound of the general formula

$$Ti(OR^3)_3 \qquad (VI)$$

in which $R^3$ has the same meaning as in formula (V), and an alkoxysilane of the general formula

$$R^1\text{-}Si(OR^3)_3 \qquad (VII)$$

in which $R^1$ has the same meaning as in formula (IV) and $R^3$ as in formula (V) and/or alkoxysilane of the general formula

$$R^2{}_2Si(OR^3)_2 \qquad (VIII)$$

in which $R^2$ has the same meaning as in formula (IV) and $R^3$ has the same meaning in formulae (VII) and (VIII) as in formulae (V) and (VI), in a largely water-miscible solvent, which does hewever dissolve the compounds according to formulae (V) to (VIII), and adding to the solution while stirring a quantity of water at least sufficient for complete hydrolysis and condensatoin, the precondensing the reaction mixture with continued sterring at a specific temperature in the range from room temperature to 200°C,
stirring the solid which is forming, optinally after the addition of further solvent or water, from a further 1 hour to 6 hours at 60° to 200°C at standard pressure or a pressure corresponding to the sum of partial pressures at the temperature concerned,

c) than separating the heterosiloxane formed from liquid phase using usual techniques, optionally washing,

d) then treating in water, in a water/alcohol mixture or in pure alcohol in the presesence of an acid or a basic catalyst, and

e) optionally washing, drying at room temperature to 200°C, optionally under a protective gas atmosphere

or a vacuum, optionally conditioning for 1 to 100 hours at temperatures of 150° to 300°C under a protective gas atmosphere or a vacuum, optionally grinding and/or classifying.

B) in the form of a block copolycondensate by precodensing the monomeric components according to formulae (V), (VI), (VII) and (VIII), each mutually independently, with or without the use of a solvent which dissolves the starting materials, preferably a linear or branched alcohol with 1 to 5 C atoms corresponding to the alkoxy groups, in the presence of a quantity of water which is insufficient for complete hydrolysis, preferably from 1 to 100 mol% of the quantity required, over a period of 5 minutes to 5 days at room temperature to 200°C, combining the precondensates obtained and then, after the addition of further water and/or further solvent, performing complete hydrolysis and polycondensation and measures according to A) and steps b) to e) or

C) in the form of mixed copolycondensate by precondensing at least one, but at most three, of the monomeric components according to formulae (V), (VI), (VII) and (VIII), mutually indepently, according to B), combining the precondensate or the precondensates obtained and at least one of the non-precondensed components and performing the measures according to A) and steps b) to e) characterised in that the post-treating is performed in autoclave over a period of 1 hour to 5 days at temperatures of 60°C to 250°C under a pressure corresponding to the sum of partial pressure at the temperature concerned.

2. Dental material according to claim 1,
characterised in that
it contains 40 to 90 wt,-% of finely divided heterosiloxane filler.

3. Dental material according to claim 1,
characterised in that
the finely divided heterosiloxane filler has a specific surface area of about 0 to 100 $m^2/g$ and a particle size of 0,1 µm to 30 µm.

4. Dental material according to claim 1,
characterised in that
a heterosiloxane is used as the filler, which heterosiloxane consists of units of the formulae (I), (II) and special units of the formula (IV)

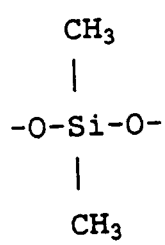

wherein the molar ratio of the sum of the units formulae (I) and (IV) to the units of formula (II) is 1:1 to 100:1.

5. Dental material according to claim 1,
characterised in that
the random copolycondensate may be obtained by precondensing the monomeric components according to formulae (V), (VI), (VII) and (VIII) with or without the use of a solvent which dissolves the starting materials, preferably linear of branched alcohol with 1 to 5 C atoms corresponding to the alkoxy groups, in the presence of a quantity of water which is insufficient for complete hydrolysis, preferably from 1 to 100 mol% of the quantity required, over a period of 5 minutes to 5 days at room temperature to 200°C.

6. Dental material according claim 1,
characterised in that
its filler may be formed in the form of a block copolycondensate and a mixed copolycondensate by dissolving the starting materials using a linear or a branched alcohol with 1 to 5 C-atoms corresponding to the alkoxy groups of the starting materials.

**7.** Dental material according to claim 1 - 3,
characterised in that
the random copolycondensate may be obtained by performing hydrolysis and condensation in methanol, ethanol, n- and i-propanol, n- and i- butanol or p-pentanol.

**8.** Dental material according to claim 1,
characterised in that
according to step a) water is added in an amount of 1 to 100 mol% of the amount sufficient for complete hydrolysis to obtain the block copolymerisate.

**9.** Dental material according to claim 1,
characterised in that
its filler may be obtained by using for precondensation catalyst which is acid, basic and/or contains metal.

**10.** Dental material according to claim 1,
characterised in that
ammonia is used as basic catalyst for post-condensation.

**11.** Application of the dental material according to the preceding claims for the preparation of inlays, overlays, crowns, bridges, dental, prostheses, artificial teeth and adhesives for the attachment of inlays, crowns and bridges.

**Revendications**

**1.** Matériau dentaire durcissable obtenu à partir d'une masse pâteuse pour donner une masse très brillante polissable, a base d'un liant organique polymèrisable qu'il contient au moins un monomère de methylacrylate ou acrylate polymerisable et presentent une surface spècifique de environ 0 à 200 $m^2/g$ et un taille de particules de 0,01 µm à 100 µm, qui est constitué d'unités de formule:

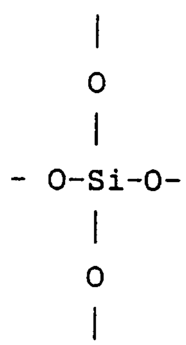

(I)

et d'untités de formule:

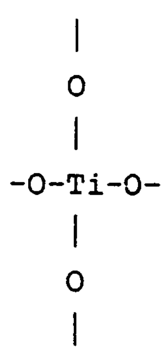

(II)

et peut contenir en outre des unités de formule:

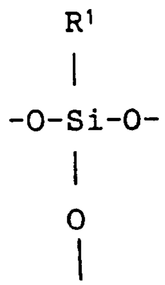

(III)

dans laquelle $R^1$ est un groupe alkyle linéaire ou ramifié avec 1 à 18 atomes de C, un groupe phenyle ou un groupe arylalkyle ou un groupe alkyle lié à un reste acrylate ou methacrylate linéaire, ramifié le cas echeant avec 1 à 6 atomes de C, ou un groupe à insaturation olèfinique simple, de préférence un reste hydrocarbure à insaturation en fin de chaîne, linéaire, le cas échéant ramifié avec 2 à 8 atomes de C, ou un reste hydrocarbure cyclique, à simple insaturation oléfinique avec 5 - 8 atemes de C, et les cas échéant des unités de formule:

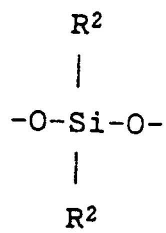

(IV)

dans laquelle $R^2$ est un groupe méthyle, éthyle ou phényle, et les valences libres des atomes d'oxygène fixés sur les atomes de silicium ou de titane dans les unités (I) à (IV) comme dans les réseaux silice peuvent être saturées par un atome de silicium d'une unité identique ou différente et/ou par un atome de titane, la proportion de la somme des atomes de siliciumdes unités de formule (I), (III) et (IV) aux atomes de titane des unités de formule (II) est de 1:1 à 100:1 et la proportion des atomes de silicium des unités (I) à la quantité totale des atomes de silicium représente 100 % molaire à 30 % molaire,

A) qu'on peut obtenir la charge qui y est contenue sons forme d'un

a) copolycondensat statistique, en faisant réagir un alcoxysilane de formule générale:

$$Si(OR^3)_4 \qquad (V)$$

dans laquelle $R^3$ est un groupe alkyle linéaire ou ramifié avec 1 à 5 atomes de C, et un composé de titane de formule générale:

$$Ti(OR^3)_4 \qquad (VI)$$

dans laquelle $R^3$ a la même signification qu'a la formule (V) et le cas échéant un alcoxysilane de formule générale:

$$R^1 - Si(OR^3)_3 \qquad (VII)$$

das laquelle $R^1$ a la même signification qu'a la formule (IV) et $R^3$ qu'a la formule (V) et le cas échéant un alcolxysilane de formule générale:

$$R^2{}_2Si(OR^3)_2 \qquad (VIII)$$

dans laquelle $R^2$ a la même signification qu'a la formule (IV) et $R^3$ dans les formules (VII) et (VIII) a la même signification que dans les formules (V) et (VI), dans un solvant largement miscible a l'eau mais dissolvant les composés de formules (V) à (VIII), et on ajoute à la solution sous agitation une quantité au moins nécessaire à l'hydrolyse complète et à la condensation, puis on précondense le mélange réactionnel en continuant d'agiter à une température définie dans l'intervalle compris entre la température ambiante et 200°C,

b) on agite le solide qui se forme le cas échéant après addition de solvant supplémentaire ou d'eau pendant encore 1 heure à 6 heures, entre 60° et 200°C, sous pression atmosphérique ou sous une pression, qui correspond à la somme des pressions partielles à la pérature correspondante.

c) puis on sèpare de la phase liquide selon les technique habituelles,

d) en traitant la charge hétérosiloxane lavée dans un mélange eau/alcohol ou dans l'alcohol pur, en présence d'un catalysateur acide ou basique, et

e) le cas échéant on lave, entre la température ambiante et 200°C, le cas échéant sous atmosphère de gaz protecteur ou on sèche sous vide,
le cas échéant on broie et/ou on classe.

B) qu'on peut optenir sa charge sous forme d'un blocpolycondensat en précondensat les composants monomères de formules (V), (VI), (VII) et (VIII), indépendamment les unes des autres, en utilisant ou non un solvant dissolvant les produits de départ, de préférence un alcool linéaire ou ramifié correspondant aux groupes alcoxy avec 1 à 5 atomes de C, en présence d'une quantité d'eau insuffisante pour realiser l'hydrolyse complète, de préférece de 1 à 100 % de la quantité molaire qui serait nécessaire à cela, sur une période de 5 minutes à 5 jours à température comprise entre la température ambiante et 200°C, on purifie les produits condensés obtenus et ensuite, après addition de solvan supplémentaire on réailise l'hydrolyse complète et la polycondensation selon la revendication 7.

C) qu'on peut obtenir sa charge en precondensat les composants monomères de formule (V), (VI), (VII) et (VIII) au moins un monomère mais au maximum 3 monomères au plus indépendamment les unes des autres, et on mèlange le ou les produits précondensés obtenus et au moins un composant non précondensé et après on réalise le mesure selon A, charactérisé en ce qu'on réalise l'hydrolyse complète et la polycondensation autoklave sur une periode de 5 minutes à 5 jours à des températures compuises entre 60°C et 250°C sous une pression, qui est la somme des pression partielles correspondant à chque temperature.

**2.** Matériau dentaire selon revendication 1,
charactérisé en ce que les chages d'hétérosiloxane sout utilisées à une concentration de 40 à 90 en poids darus le matériau dentaire.

**3.** Matériau dentaire selon la revendication 1,
charactérisé en ce que les chages d'hétérosiloxane présentent une surface spécifique de environ à100 $m^2/g$ et une taille de particules de 0,1 µm à 30 µm.

**4.** Matériau dentaire selon revendication 1,
charactérisé en ce qu'on utilise comme charge un hétérosiloxane, qui est constitué des unités de formule (I), (II) et des unités spéciales de formule (IV) :

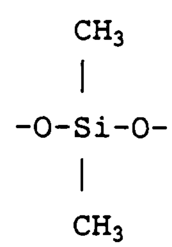

la proportion moléaire de la somme des unités de formule (I) et (IV) aux unités de formule (II) étant de 1:1 à 100:1.

**5.** Matériau dentaire selon revendication 1, charactérisé en ce qu'on peut obtenir d'un copolycondensat statistique en précondensant les composants monomères de formules (V), (VI), (VII) et (VIII), en utilisant ou non l'un des solvants dissolvant les produits départ, de préférence un alcool linéaire ou ramifié correspondant aux groupes alcoxyavec 1 à 5 atomes de C, en présence d'une quantité d'eau insuffisante pour réaliser l'hydrolyse complète, de préférence de 1 à 100 % de la quantité molaire qui serait nécessaire à température comprise entre la température ambiante et 200°C.

**6.** Matériau dentaire selon la revendication 1, charactérisé en ce qu'on peut obtenir sa charge sous forme d'un compolycondensat mixte ou d'un bloccopolycondensat, en utilisant un solvant dissolvant les produits de départ, de préférence un alcool linéaire ou ramifié correspondant aux groupes alcoxy avec de 1 à 5 atomes de C.

**7.** Matériau dentaire selon lesrevendication 1 à 3, charactérisé en ce qu'on peut optenir sa charge en réalisant l'hydrolyse et la condensation dans le méthatnol, éthanol, n-et i-propanol. n-et i-butanol ou n-pentanol.

**8.** Matériau dentaire selon la revendication 1, charactérisé en ce que les charges de bloccopolycondensat sout constituées en présence de 1 à 100 % de la quantité molaire d'eau qui serait necessaire pour réaliser l'hydrolyse complète.

**9.** Matériau dentaire selon la revendication 1, charactérisé en ce qu'on peut obtenir sa charge, en utilisant pour la précondensation un catalyseur de condensation acide, basique et/ou centenant un métal.

**10.** Matériau dentaire selon la revendication 1, charactérisé en ce qu'on peut obtenir sa charge, en utilisant pour la post-condensation ammoniaque.

**11.** Utilisation du matériau dentaire selon les revendications précédentes pour produire des inlays, couronnes, bridges, prothèses dentaires, dents artificielles et adhésifs pour fixer les inlays, couronnes et bridges.